# EUROPEAN PATENT SPECIFICATION

(11) **EP 1 734 992 B1**
(45) Date of publication and mention of the grant of the patent: **28.09.2011**
(21) Application number: 05705920.6
(22) Date of filing: 19.01.2005
(51) Int. Cl.: A61K 39/125, C12Q 1/70

(54) **IMPROVED INACTIVATED FCV VACCINES**
VERBESSERTE INAKTIVIERTE FCV-VAKZINEN
VACCINS AMELIORES A FCV INACTIVE

(30) Priority: 21.01.2004 US 537849 P
(43) Date of publication of application: 27.12.2006
(73) Proprietor: Merial Limited, Duluth GA 30096-4640 (US)
(72) Inventor: POULET, Hervé, F-69110 Sainte Foy lès-Lyon (FR); BARRAL, Denis, F-38540 Heyrieux (FR)
(74) Representative: Harding, Charles Thomas
(86) International application number: PCT/US2005/001721
(87) International publication number: WO 2005/072214

(56) References cited:
- WO-A-01/46390
- US-B1- 6 355 246
- US-B1- 6 534 066
- US-B1- 6 534 066

## Description

### FIELD OF THE INVENTION

The present invention relates to improved inactivated and stabilized feline calicivirus (FCV) immunogenic compositions. The invention also provides a process for producing inactivated and stabilized FCV, and the use of such inactivated and stabilized FCV in the production of FCV immunogenic compositions. The invention further provides the immunogenic compositions according to the invention for use in methods of inducing immune responses in animals of the Felidae family, preferably a cat.

### BACKGROUND OF THE INVENTION

Feline calicivirus (FCV) was first described in 1957 (Fastier, L.B. (1957) Am. J. Vet. Res. 18: 382-389). FCV affects a large number of animals of the Felidae family, with FCV being carried by up to 15 to 20% of clinically healthy domestic cats (Coutts et al (1994) Vet. Rec. 135: 555-556; Ellis, T.M. (1981) Australian Vet. J. 57: 115-118; Harbour et al. (1991) Vet. Rec. 128: 77-80; Reubel et al, (1992) Feline Dentistry 22: 1347-1360). Calicivirus often occurs with another upper respiratory infection, such as feline herpes virus (FHV), rhinotracheitis virus, or chlamydiosis in domestic cats and wild felids. FCV is transmitted horizontally and there is no evidence of vertical transmission from the mother to its kitten during gestation (Johnson, R.P. (1984) Res. Vet. Sci. 31: 114-119). FCV transmission occurs primarily through contact between infected animals and healthy animals or by the airways during sneezing (Wardley R C. (1976) Arch. Virol 52: 243-249). FCV is quite resistant to a number of disinfecting agents and thus can also be spread in the absence of direct contact.

FCV is generally known to cause disease characterized by conjunctivitis, rhinitis, tracheitis, pneumonia and by vesicularization/ulceration of the epithelium of the oral cavity. Other symptoms include fever, anorexia, lethargy, stiff gait, and sometimes nasal and ocular discharge. FCV usually affects the throat, and sometimes the lungs; it can also infect the intestines and has been isolated from feces. After an initial phase of hyperthermia, these respiratory diseases are generally followed by buccal ulcerations (palate, tongue, lips, and nose), rhinitis, conjunctivitis, and possibly anorexia and asthenia. FCV can also cause pneumonia, enteritis, and articular pain (lameness syndrome). Morbidity can be high and recovery is followed by a prolonged carrier state. The type of disease symptoms caused by FCV depends on the FCV strain; some strains will produce little or no disease, while other more virulent strains cause pyrexia, anorexia, depression or pneumonia. One particular strain can cause ulcers on the paws as well as in the mouth.

Feline calicivirus of the *Caliciviridae* familly is a non-enveloped virus, comprising a single-stranded positive-sense RNA genome that is polyadenylated and is about 7.7 kilobases in size (Radford et al. (1997) Proc. 1st Int. Symp. Caliciviruses ESVV 93-99). The FCV capsid is comprised of a single major capsidal protein of 66 kDa (kilodalton), the p66 protein. The molecular biology of the caliciviruses was reviewed by Clarke and Lambden (1997) J. Gen. Virol. 8: 291-301. Like many RNA viruses, large heterogeneity exists within the viral population of FCV. The antigenic variations, demonstrated since the beginning of the 1970s by cross-serum neutralization experiments, make it possible to classify the FCVs into several viral strains or quasi-species (Radford et al. (1997) Proc. 1st Int. Symp. Caliciviruses ESVV 93-99). Several FCV strains have been identified and isolated, in particular strain F9 (deposited with the American Type Culture Collection or ATCC under the accession number VR-782), strain 2280 (ATCC VR-2057), strain KCD (ATCC VR-651), strain CFI (ATCC VR-654), strain FCV-LLK and strain FCV-M8.

Due to the long carrier state after recovery from an FCV infection, as well as FCV resistance to disinfecting agents, FCV is highly contagious and easily spread, particularly among groups of animals in close proximity, for example, in animal shelters or veterinary clinics. Therefore, there has been and remains a strong need in the art for effective FCV vaccines.

Vaccination against FCV was introduced at the end of the 1970s, using attenuated FCV strains, primarily strain F9 isolated in the USA in 1958 by Bittle (Bittle et al. (1960) Am. J. Vet. Res. 21: 547-550) or strains derived from F9 by passage *in vitro* or *in vivo* ("F9-like").

Vaccines that are based on inactivated FCV strains are also available. These vaccines mainly use strains 255 and 2280, which were isolated in the USA in 1970 in a cat with pneumonia (Kahn and Gillepsie, (1970) Cornell Vet. 60: 669-683; Povey et al. (1980) J. Am. Vet. Med. Assoc. 177: 347-350) and in 1983 in a cat suffering from lameness, respectively (Pedersen et al. (1983) Fel. Prac. 13: 26-35; Pedersen N.C. and Hawkins K.F. (1995) Vet Microbiol. 47: 141-156).

Because of antigenic drift over time, antisera produced against vaccine strains isolated in the 1960's and 1970s, such as strains F9, 255 or 2280, neutralize only a few isolates of the strains isolated in the 1990s. For example, the anti-F9 serum neutralizes 43% of the American isolates of the period 1990-1996, in contrast to its ability to neutralize 56% of the isolates of the period 1980-89 and 86% of the isolated of the period 1958-79, and only 10% of the English isolates of the period 1990-96 (Lauritzen et al. (1997) Vet. Microbiol. 56: 55-63). Accordingly, attenuated and inactivated vaccines from old FCV strains at present no longer offer sufficient protection against recently isolated FCV strains.

US Patent No. 6,534,066 describes the use of new strains of FCV for the production of FCV vaccines. Among these strains, FCV strain 431 (deposited at the CNMC under the accession number I-2166) is an isolate the antiserum for which was shown to neutralize a large number of heterologous field isolates. This is in contrast to the historical vaccine strains, such as F9 and FCV-225, whose antisera neutralize very limited numbers of recent field FCV isolates.

The majority of the commercial FCV vaccines are attenuated vaccines. Only few inactivated vaccines are available, all of them containing an adjuvant. Povey and coworkers (Povey et al. (1978) Feline Practice 8(3): 35-42) describe a formalin inactivated and adjuvanted FCV preparation used in kittens.

When a vaccine is inactivated, the inactivation is usually performed by chemical treatment with agents such as formalin, or formaldehyde, β-propiolactone, ethyleneimine, binary ethyleneimine in the presence or absence of heat treatment. In U.S. Patent No. 6,534,066, for example, FCV is inactivated by ethyleneimine and adjuvanted with an oil-in-water emulsion. U.S. Patent No. 6,355,246 describes attenuated FCV vaccines isolated from feline urine. Inactivation of FCV can be achieved by treatment with formaldehyde or binary ethyleneimine (BEI). Notably, U.S. Patent No. 6,355,246 does not direct or provide the skilled artisan with methods of inactivating FCV.

The inactivated vaccines heretofore used against FCV require or prefer adjuvants in the vaccine or immunogenic composition to improve the immune response and to induce better protection against heterologous FCV strains emerging in the cat population. However adjuvanted vaccines induce a higher rate of local adverse reactions than non-adjuvanted ones (Gobar et al., (2002) JAVMA 220(10): 1477-1482) and thereby increase the risk of vaccine-associated fibrosarcomas at the injection site (Baker R.J. (1998) Feline Practice 26(5): 18-20).

Presently, non-adjuvanted FCV vaccines are modified live vaccines, usually containing the F9 strain. The residual virulence of FCV F9 has been demonstrated by several studies in post-vaccinal calicivirosis (Dawson et al., (1993) Vet. Rec. 132: 346-350). Although only one FCV serotype exists, high antigenic variation between FCV isolates has been observed and new field isolates are regularly identified (Lauritzen, et al. (1997) Vet. Microbiol. 56: 55-63). This high antigenic variation often leads to increased failure rates of FCV neutralization by antisera based on an F9 vaccine. Furthermore, FCV modified live strains are implicated in the emergence of new antigenic variants in the field (Radford et al., (1997) Vaccine 15(12/13): 1451-1458). The safety of these modified live vaccines is therefore questionable.

Consequently, there remains a need in the art for efficacious, inactivated non-adjuvanted FCV vaccines or immunogenic compositions with improved safety and which are capable of inducing a strong immune response against heterologous FCV strains.

### SUMMARY OF THE INVENTION

Previous inactivated FCV vaccines or immunogenic compositions usually comprise a mixture of FCV virions and protein fractions resulting from the degradation of the viral capsid. The present inventors have found that for a non-adjuvanted vaccine or immunogenic composition, it is essential to limit the degradation of the viral capsid and to retain intact virions as much as possible.

It has been surprisingly found that subjecting FCV to treatment by an inactivating agent that inactivates the virus and to treatment with formaldehyde that may stabilize the virion allows one to obtain an inactivated FCV composition having good efficacy, even in the absence of any adjuvant. Without wanting to be bound to any particular theory, it is believed that the presence of formaldehyde stabilizes the viral capsids. The increased stability of the viral capsid results in enhanced stability of the FCV vaccine or immunogenic composition during long-term storage and before administration to the animal. Other chemical compounds acting similarly to stabilize the viral capsid may be used in place of formaldehyde.

A first aspect of the present invention provides an inactivated stabilized, non-adjuvanted immunogenic composition against feline calicivirus (FCV), comprising an FCV inactivated by one or more inactivating agents and stabilized by an aldehyde compound, wherein the aldehyde compound comprises a linear C1-C5 alkyl chain, and wherein the immunogenic composition is in admixture with an acceptable vehicle or excipient, and wherein the inactivating agents are selected from the group consisting of ethyleneimine, acetylethyleneimine, propyleneimine, and β-propiolactone.

Preferably, the excipient or vehicle is veterinarily acceptable. In another embodiment, the immunogenic composition is freeze-dried and is in admixture with a freeze-drying excipient or vehicle.

Preferably, the inactivating agent! is ethyleneimine.

When ethyleneimine is used as the inactivating agent, the ethyleneimine is present from about 0.5 mM to about 20 mM, preferably from about 1 mM to about 10 mM.

When the aldehyde compound comprises a linear C1 alkyl chain, the aldehyde compound comprises one aldehyde group. When the aldehyde compound comprises a linear C2-C5 alkyl chain, the aldehyde compound comprises two aldehyde groups. In an alternative embodiment, one of the two aldehyde groups can be replaced by a ketone or an epoxy group. The aldehyde compound can be selected from the group consisting of formaldehyde, glycidaldehyde, glutaraldehyde, glyoxal, or methylglyoxal. When the aldehyde compound is formaldehyde, the formaldehyde is present from about 0.05 g/l to about 0.8 g/l. Preferably, the formaldehyde is present from about 0.1 g/l to about 0.5 g/l.

A further embodiment further comprises a neutralizing compound comprising thiol groups (e.g. thiosulfate and cysteine). The present description also comprises at least one FCV strain, wherein at least one or all of the FCV strains are inactivated and stabilized. The FCV strain can be selected from the group consisting of FCV F9, FCV 255, FCV 2280, FCV 431, FCV G1, FCV LLK, FCV KCD, FCV CFI, FCV M8, and FCV US 100869 (ATCC Accession No. FCV PTA 5930).

In another embodiment, the immunogenic compositions of the invention further comprise at least one non-FCV immunogen from a feline pathogen, which can be selected from the group consisting of feline herpesvirus (FHV), feline leukemia virus (FeLV), feline panleukopenia virus (FPV), feline infectious peritonitis virus (FIPV), feline immunodeficiency virus (FIV), rabies virus, and Chlamydia. Preferably, the at least one non-FCV immunogen from a feline pathogen comprises a live attenuated microorganism, or a recombinant vector that expresses at least one non-FCV immunogen from a feline pathogen.

A second aspect of the invention provides a process for inactivating and stabilizing FCV, comprising the steps of reacting FCV with an inactivating agent and an aldehyde compound, wherein the aldehyde compound comprises a linear C1-C5 alkyl chain, and wherein the inactivating agents are selected from the group consisting of ethyleneimine, acetylethyleneimine, propyleneimine, and β-propiolactone, and recovering the inactivated and stabilized FCV. A preferred embodiment recovers the inactivated and stabilized FCV by size exclusion chromatography, ultracentrifugation, and selective precipitation.

In another aspect of the present invention, a process for producing an inactivated, stabilized, non-adjuvanted immunogenic composition, against FCV for long-term storage is provided, comprising mixing the inactivated and stabilized FCV of the invention in an amount sufficient to induce an immune response to FCV with a freeze-drying excipient, and freezing the composition.

Yet another aspect provides a process for producing an inactivated, stabilized, non-adjuvanted immunogenic composition against FCV, comprising mixing the inactivated and stabilized FCV of the description in an amount sufficient to induce an immune response to FCV with a veterinarily acceptable excipient or vehicle.

Also provided in the present disclosure are the inactivated, stabilized, non-adjuvanted immunogenic compositions of the invention, for use in inducing an immune response in a Felidae against FCV.

A further aspect provides the inactivated, stabilized, non-adjuvanted immunogenic composition of the invention, and at least one non-FCV immunogen from another feline pathogen, for use in inducing an immune response in a Felidae against FCV and at least one non-FCV immunogen from a feline pathogen.

### BRIEF DESCRIPTION OF THE DRAWINGS

The following Detailed Description, given by way of example, and not intended to limit the invention to specific embodiments described, may be understood in conjunction with the accompanying Figures, in which:
Figure 1 is a graph showing the mean of the total clinical score during the two weeks post-challenge per type of composition administered (according to Example 3).
Figure 2 is a graph showing the mean of the maximum clinical score for an observation on a cat during the two weeks post-challenge per type of composition administered (according to Example 3).
Figure 3 is a graph showing the mean anti-FCV255 neutralizing antibody titers (expressed as log₁₀) per group and day (according to Example 4).
Figure 4 is a graph showing the global scores per group (according to Example 4).
Figure 5 is a graph showing the mean FCV excretion after challenge per group and day, expressed as log₁₀ CCDE)₅₀/ml (according to Example 4).

### DETAILED DESCRIPTION OF THE INVENTION

It is noted that in this disclosure and particularly in the claims, terms such as "comprises", "comprised", "comprising" and the like can have the meaning attributed to it in U.S. Patent law; e.g., they can mean "includes", "included", "including", and the like; and that terms such as "consisting essentially of' and "consists essentially of" have the meaning ascribed to them in U.S. Patent law, e.g., they allow for elements not explicitly recited, but exclude elements that are found in the prior art or that affect a basic or novel characteristic of the invention.

The term "immunogenic composition" covers any composition capable, once administered to the target species under the conditions of the invention, of inducing an immune response directed against FCV. The term "vaccine" is intended to mean a composition capable of inducing effective protection. The target species are the Felidae, preferably cats.

The present invention relates to an inactivated, stabilized, non-adjuvanted FCV immunogenic composition or vaccine comprising FCV that has been subjected to an inactivating agent and to a stabilizing aldehyde compound. A preferred group of stabilizing compounds are aldehydes which have a linear alkyl C1-C5 chain comprising one aldehyde group when the chain is C1 and two terminal aldehyde groups when the chain is C2-C5, and optionally one aldehyde group may be replaced by a ketone or an epoxy group when the chain is the C2-C5 chain, and the immunogenic composition or vaccine is either freeze-dried in admixture with a freeze drying excipient or vehicle, or in admixture with a veterinarily acceptable excipient or vehicle.

An "inactivating agent" is an agent able to block the multiplication of a virus by an irreversible reaction, mainly, but not limited to, reactions with viral nucleic acids, which does not substantially affect the immunogenic property of the virus. The inactivating agents of the invention are ethyleneimine, acetylethyleneimine, propyleneimine, and β-propiolactone. In a preferred embodiment, the inactivating agent is ethyleneimine.

In a preferred embodiment, the FCV is inactivated by ethyleneimine. The final concentration of ethyleneimine can be from about 0.5 mM to about 20 mM, and preferably from about 1 mM to about 10 mM. The temperature can be from about 2°C to about 40°C, and preferably from about 5°C to about 30°C.

The stabilizing aldehyde compounds can react with amino groups (e.g. amino groups on lysine, arginine or histidine amino acids) and hydroxyl groups of protein(s) (e.g. hydroxyl groups on tyrosine amino acids) and can form linkages between two proteins and/or within a protein. The stabilizing aldehyde compound is preferably selected from the group consisting of formaldehyde (or methanal), glycidaldehyde (or 2,3-epoxy-1-propanal), glutaraldehyde (or 1,5-dial-pentane), glyoxal (or 1,2-dial-ethane), methylglyoxal (or pyruvaldehyde). In a preferred embodiment, the stabilizing aldehyde compound is formaldehyde.

When formaldehyde is used as the stabilizing aldehyde, the final concentration may be from about 0.05 g/l to about 0.8 g/l, preferably from about 0.075 g/l to about 0.6 g/l, and more preferably from about 0.1 g/l to about 0.5 g/l. The temperature may be from about 2°C to about 37°C, preferably from about 2°C to about 22°C, and more preferably from about 4°C to about 7°C.

To adjust the stabilization conditions (temperature, concentration of the stabilizing aldehyde compound and duration), quantification of the FCV virions may be performed. Any appropriate technique to quantify virions may be used, for example, an enzyme-linked immunosorbent assay (ELISA) using a monoclonal or polyclonal antibody specific for the FCV capsid protein. Before ELISA quantification, the virions can be separated from the treated viral culture with techniques known by the skilled artisan, for example size exclusion chromatography, ultracentrifugation on a sucrose gradient, ultracentrifugation on a cesium chloride gradient, and selective precipitation, for example, polyethylene glycol (PEG) precipitation.

The FCV suspension used for the immunogenic composition or vaccine contains preferably from about 10^{8.5} to about 10¹¹ CCID₅₀ per dose before inactivation, more preferably from about 10⁹ to about 10¹⁰ CCID₅₀ per dose before inactivation. After completion of inactivation and/or stabilization, the inactivating agent and/or the stabilizing aldehyde compound can be removed from the suspension by neutralization methods known by one skilled in the art, for example, by adding neutralizing compounds comprising thiol groups (e.g, thiosulfate, cysteine).

Elimination of the inactivating agent and/or the stabilizing aldehyde compound, or alternatively, recovering the inactivated and stabilized FCV from the suspension can be achieved by techniques known by one skilled in the art, for example, size exclusion chromatography, ultracentrifugation on a sucrose gradient, ultracentrifugation on a cesium chloride gradient, selective precipitation for example polyethylene glycol (PEG) precipitation.

Recovery of the virions from the suspension, after virus inactivation and stabilization, can be achieved concomitantly in the processing step that eliminates the inactivating agent and/or the stabilizing aldehyde. In an alternative embodiment, recovering the inactivated and stabilized virions may be achieved in a discrete step that is not concomitant with the step that eliminates the inactivating agent and/or the aldehyde compound. This step of virion recovery may be achieved by techniques known by one skilled in the art, for example, size exclusion chromatography, ultracentrifugation on a sucrose gradient, ultracentrifugation on a cesium chloride gradient, and selective precipitation, for example, polyethylene glycol (PEG) precipitation.

The immunogenic compositions and vaccines according to the description may comprise a combination of at least two FCV strains that are inactivated and stabilized, or a combination of at least two FCV strains wherein at least one of the FCV strains is inactivated and stabilized.

Preferably, the FCV strain(s) is/are selected from those recently isolated from the field. Preferred strains include the strains 431 (deposited at the CNCM under the accession number I-2166; or any strain reacting with the monoclonal antibody 44 secreted by the hybridoma deposited at the CNCM under the accession number 1-2282; see U.S. Patent No. 6,534,066), FCV G1 (deposited at the CNCM under the accession number I-2167) (CNCM = "Collection Nationale de Cultures de Microorganismes", Pasteur Institute, Paris, France), FCV US 100869 (also known as FCV PTA 5930; deposited at the ATCC on April 22, 2004) (ATCC = "American Type Culture Collection", Manassas, Virginia, USA) and more generally any of the new highly virulent strains described in publications (Pedersen et al. (2000) Vet Microbiol. 73 : 281-300; Schorr-Evans et al. (2003) JFMS 5 : 217-226; Hurley et al. (2003) Vet Clin. Small Anim. 33: 759-772). In a preferred embodiment, the immunogenic composition or vaccine comprises inactivated and stabilized FCV 431 (or any strain reacting with the monoclonal antibody 44) and inactivated and stabilized FCV G1. In another preferred embodiment the immunogenic composition or vaccine comprises inactivated and stabilized FCV US 100869 (ATCC Accession No. FCV PTA 5930). Other strains of FCY that can be used in or in addition to the present description include, but are not limited to, FCV F9, FCV 255, FCV 2280, FCV LLK FCV KCD, FCV CFI, and FCV M8.

The inactivated and stabilized FCV immunogenic composition or vaccine can be easily combined with live attenuated or inactivated vaccine(s) or immunogenic compositions that is/are used for other feline disease(s). Therefore, another object of the description is a non adjuvanted combination immunogenic composition or vaccine comprising at least one stabilized and inactivated FCV and at least one non-FCV immunogenic component for inducing in the host an immune response against at least one other feline pathogen, wherein said non-FCV immunogenic component may be an immunogen from another feline pathogen or a recombinant vector expressing this immunogen, wherein the non-adjuvanted combined immunogenic composition or vaccine is either in a freeze-dried in admixture with a freeze-drying excipient or vehicle or in admixture with a veterinarily acceptable vehicle or excipient The freeze-dried form is preferred.

In a preferred embodiment, the non-adjuvanted combined immunogenic composition or vaccine comprises the non-FCV immunogenic component either in the form of a live attenuated microorganism or of a recombinant vector expressing at least one immunogen from the feline pathogen. The recombinant vector may be a plasmid or a viral vector; for example the vector can be a poxvirus, an adenovirus, or a herpesvirus. The freeze-dried form is preferred.

The additional non-FCV feline pathogens are preferably selected from the group comprising the feline rhinotracheitis virus or feline herpesvirus (FHV), the feline leukemia virus (FeLV), the feline panleukopenia virus or feline parvovirus (FPV), the feline infectious peritonitis virus (FIPV), the feline immunodeficiency virus (FIV), the rabies virus, and Chlamydia (e.g. *Chlamydophila felis*).

The combined immunogenic composition or vaccine in sofar as the invention is concerned combines at least the FCV immunogenic composition of claim 1 in addition to non-FCV immunogenic components such as:
- FHV, FPV, FeLV and Chlamydia
- FHV, FPV and FeLV
- FHV, FPV and Rabies
- FHV, FPV and Chlamydia
- FHV, FPV, Chlamydia and Rabies
- FHV and FPV
- FHV and Chlamydia
- FHV

In a preferred embodiment of these various combinations, attenuated live microorganisms are used for FHV, FPV and Chlamydia and a recombinant vector(s) expressing FeLV genes is/are used for FeLV. The recombinant vector may be a canarypox virus (for example vCP97 as described in U.S. Patent No. 5,753,103) that expresses *env* and *gag*/*pol* FeLV genes. For rabies, a recombinant vector may be used, notably a canarypox virus (for example vCP65 as described in U.S. Patent No. 5.843.456) that expresses G glycoprotein rabies gene.

Another object of the invention is a process of inactivating and stabilizing FCV, comprising reacting FCV with an inactivating agent and a stabilizing aldehyde compound formed of a linear alkyl C1-C5 chain comprising one aldehyde group when the chain is C1 and two terminal aldehyde groups when the chain is C2-C5, and optionally one aldehyde group may be replaced by a ketone or an epoxy group when the chain is the C2-C5 chain. Preferred embodiments for the inactivating agent and the stabilizing aldehyde compound and their conditions of use are as described above, insofar as the invention is concerned.

The process of the invention comprises the culturing of FCV in suitable host cells, the treatment with the inactivating agent and the stabilizing aldehyde compound. The culture and propagation of the FCV virus is preferably carried out on feline cells, more particularly on Crandell-Reese Feline Kidney or CRFK cells (accessible from the American Type Culture Collection under the number CCL-94) with a multiplicity of infection (MOI) of 2 to 0.01 cell culture infectious doses 50% (CCID₅₀) per cell, preferably 0.5 CCID₅₀/cell.

The addition of the stabilizing aldehyde compound can be done before, during or after the inactivation step. Neutralization of the inactivating agent and/or the stabilizing aldehyde compound may be performed as described above.

The stabilized inactivated FCV virions may be concentrated and/or recovered from the suspension by conventional concentration techniques, for example by ultrafiltration and then optionally purified by conventional purification means, for example size exclusion chromatography, ultracentrifugation on a sucrose gradient, ultracentrifugation on a cesium chloride gradient, or selective precipitation for example in the presence of polyethylene glycol (PEG).

In one embodiment, the immunogenic composition or vaccine comprises freeze-dried stabilized and inactivated FCV of the invention and a freeze-drying excipient or vehicle, which can include amino acids, e.g., glutamic acids, or carbohydrates, e.g, lactose, and mixtures thereof, e.g, SPGA (sucrose/phosphate/glutamate/albumin; see also European Patent Application Serial No 0.496.135). Inactivated and stabilized FCV may be stored long-term at about 5°C, or alternatively, frozen or freeze-dried (or lyophilized) according to techniques known to the skilled artisan.

Therefore, to produce the immunogenic FCVcompositions of the present invention, the FCV is grown in cell culture on a suitable feline cell line, i.e., CRFK cells, to titers sufficient for producing a viral suspension in sufficient amounts to produce an immunogenic composition. The FCV is harvested according to methods well known in the art. The calicivirus is then concentrated, frozen, and stored at -70°C or freeze-dried and stored at 4°C.

Still another object of the invention is a process of producing an immunogenic composition or vaccine comprising providing the inactivated and stabilized FCV of the invention in a sufficient amount to induce an immune response, and either freeze-drying said FCV (and possibly adding a freeze-drying stabilizer) or in liquid form by mixing said FCV with a veterinarily acceptable excipient or vehicle. Suitable excipients are, for example, water, isotonic solutions, saline such as, but not limited to, NaCl and phosphate buffered saline (PBS), dextrose, mannitol, sorbitol, lactose, glycerol, ethanol, or the like, and combinations thereof. In addition, if desired, the vaccine may contain minor amounts of auxiliary substances such as, but not limited to, wetting or emulsifying agents, and pH buffering agents.

The immunogenic compositions and the vaccines according to the description can comprise one or more adjuvants, but need not Preferably, the compositions of the invention comprise an inactivated and stabilized FCV that is immunogenic even in the absence of adjuvant. If desired, however, an acceptable adjuvant may be included in the compositions of the invention. The acceptable adjuvant is a hydrosoluble adjuvant. Such acceptable adjuvants may be polymers of acrylic or methacrylic acid, polymers of maleic anhydride and of an alkenyl derivative, immunostimulatory sequences (ISS), in particular oligodeoxyribonucleotide sequences having one or more non-methylated CpG motifs (Klinman D.M. et al., (1996) Proc. Natl. Acad Sci. USA 93: 2879-2883; WO-A1-98/16247), an oil-in-water emulsion, in particular the SPT emulsion described on page 147 of "Vaccine Design, The Subunit and Adjuvant Approach" edited by M. Powell, M. Newman, Plenum Press 1995, and the MF59 emulsion described on page 183 of the same work, cationic lipids containing a quaternary ammonium salt, cytokines, or combinations or mixtures thereof.

Still another object of the description is a method of immunization of an animal of the Felidae family, preferably cats, including newborns, kittens, males, females, and pregnant females, against FCV disease, the method comprising administering a non-adjuvanted inactivated and stabilized FCV immunogenic composition or vaccine according to the description.

A further object of the description is a method of immunization of an animal of the Felidae family, preferably cats, including newborns, kittens, males, females, and pregnant females against at least two feline diseases including FCV disease, the method comprising administering a non-adjuvanted combined vaccine comprising inactivated and stabilized FCV according to the description, and at least one non-FCV immunogen from another feline pathogen or recombinant vector that expresses at least one non-FCV immunogen from another feline pathogen.

Different routes may carry out the administration of the immunogenic compositions or vaccines according to the description. These include, but are not limited to, the parenteral route, oronasal, intramuscular, intradermal, subcutaneous, and mucosal (e.g. oral). The preferred routes of administration include the subcutaneous or the intramuscular injection route. Vaccine or immunogenic compositions can be administered by any means that include, but are not limited to, syringes, needleless injection devices. Needle-free injectors may be used for transdermal delivery (intradermal and subcutaneous and possibly intramuscular delivery). Using a needleless injection system, the dose volumes are determined by the volume necessary to deliver the FCV immunogenic composition and may be between 0.1 ml and 1 ml.

Using a syringe, the dose volumes of the vaccines and immunogenic compositions are generally between 0.2 and 2.0 ml, preferably about 1.0 ml. Cats may be vaccinated from about 6 weeks of age. Two or more administrations may be performed, for example 3-5 weeks apart. Preferably a boost administration may be performed, for example annually. Alternatively cats can be primed with only one injection.

The invention will now be further described by way of the following non-limiting examples.

### EXAMPLES

### Example 1: Inactivation of FCV by Formaldehyde

CRFK cells (Crandell-Reese Feline Kidney cells, accessible from the American Type Culture Collection under CCL-94) were cultured at 37°C in 2-liter roller flasks (850 cm²) with modified Eagle's medium (MEM, Gibco BRL) supplemented with 2.5% of lactalbumin hydrolysate and 5% fetal calf serum. Three hundred milliliters of a cellular suspension in MEM medium, containing about 100,000 cells/ml, were added per roller flask. After 3 days, the cell layer became confluent. The cell culture medium was then replaced with serum-free MEM and strain 431 FCV virus was added at a multiplicity of infection (MOI) of 0.5 CCID₅₀ /cell. The viral culture was maintained at 37°C for 24 to 48 hours until a cytopathic effect was obtained for the whole cellular layer. The viral suspension was harvested and then clarified on a filter having a porosity of 1.5 µm and stored at 5°C. The FCV virus titer at harvest was 8.5 +/-0.3 log₁₀ CCID₅₀/ml.

The viral suspension was inactivated with ethyleneimine at the concentration of 8 mM at 22°C for 18 hours. The ethyleneimine was prepared by dissolving 36 g of sodium hydroxide pellets in 257.5 ml of distilled water and adding 87.5 g of bromoethylamine (BEA), corresponding approximately to a 1.2 M solution of ethyleneimine. At the end of inactivation, part of the inactivated viral suspension was stabilized by addition of formaldehyde at a final concentration of 0.5 g/l and at 5°C (+/- 3°C) during 24 hours. Part of the inactivated viral suspension was kept unstabilized as a control suspension.

A portion of the inactivated and stabilized viral suspension and a portion of the control suspension were subjected to selective precipitation in the presence of polyethylene glycol (PEG). PEG 6000 was added to the suspensions at a concentration of 6%, and agitated at 5°C (+/- 3°C) for 3 hours. Suspensions were then centrifuged at about 1330 g during 90 minutes. The supernatants, containing the soluble p66 protein, and the precipitates, containing the virions, were separated and recovered. The precipitates were dissolved in sterile phosphate buffer saline (PBS) without calcium and without magnesium.

Two to three days after the end of the inactivation step, FCV p66 proteins were quantified by ELISA titration in the inactivated and stabilized viral suspension, in the control suspension, in the supernatants and in the precipitates in solution. Coating anti-FCV polyclonal antibodies on a titration plate, adding different dilutions of the inactivated and stabilized viral suspension, the control suspension, the supernatants and the precipitate solution, performed ELISA titration. The titration plate was kept at 37°C for 3 hours, followed by washing the titration plate with a washing buffer, and subsequently adding a monoclonal antibody specific for FCV p66 capsid protein coupled to a peroxidase. The titration plate was further incubated at 37°C during 1 hour, after which the plate was washed with the washing buffer. To the washed plate, 100 µl of TMB solution (5,5'-tetramethylbenzidine) per well was added at 20°C, and kept in the dark at 20°C for 30 minutes. The color development was blocked by adding 50 µl of 2M sulfuric acid per well. The ELISA titers were optically measured and expressed as log₁₀ OD₅₀ (optical density 50%), which correspond to the decimal logarithm of the dilution, giving 50% of the maximum optical density. Standard deviation of the ELISA titration is 0.07.

**Table 1: Degradation of FCV virions after inactivation**

| | Inactivation without stabilization | Inactivation and stabilization |
|---|---|---|
| Total p66 protein in the viral suspension not subjected to PEG precipitation | 2.81 | 2.36 |
| P66 protein in the supernatant (soluble p66) | 2.55 | 2.18 |
| P66 protein in the precipitate (p66 on virion) | 1.95 | 2.62 |

These results demonstrate the degradation of FCV virions after inactivation in absence of stabilization and the stabilization effect of formaldehyde.

### Example 2: Stabilizing Effect of Formaldehyde on FCV under Various Conditions

The FCV 431 strain was cultured essentially as described in Example 1.

Three methods of inactivation were tested:
1) ethyleneimine at the concentration of about 4 mM at 20°C for 24 hours,
2) ethyleneimine at the concentration of about 8 mM at 20°C for 24 hours,
3) ethyleneimine at the concentration of about 8 mM at 5°C for 24 hours.

The viral suspension was stabilized by formaldehyde at various final concentrations of 0.1 g/l- 0.5 g/l and at 5°C (+/- 3°C) during 5 days. A control suspension did not contain any formaldehyde.

The FCV p66 proteins were quantified as described in Example 1 before and after selective PEG precipitation, by ELISA titration. The ELISA titers are expressed as log₁₀ OD₅₀.

**Table 2: ELISA Titers of FCV p66 proteins**

| Formaldehyde 5 days, 5°C | **Ethyleneimine** | | | | | | | | |
|---|---|---|---|---|---|---|---|---|---|
| | 4 mM, 20°C, 24h | | | 8 mM, 20°C, 24h | | | 8 mM, 5°C, 24h | | |
| | Total of protein | Soluble p66 | p66 on virion | Total of protein | Soluble p66 | p66 on virion | Total of protein | Soluble p66 | p66 on virion |
| 0.5 g/l | 2.15 | 1.53 | 1.56 | 1.96 | 1.37 | 1.09 | 2.11 | 1.42 | 1.38 |
| 0.1 g/l | 2.38 | 1.88 | 1.56 | 2.24 | 1.87 | 0.98 | 2.27 | 1.72 | 1.35 |
| 0 g/l | 2.41 | 1.86 | 1.34 | 2.14 | 1.85 | 0.82 | 2.3 | 1.76 | 1.14 |

These results demonstrated the stabilizing effect of 0.1 g/l and 0.5 g/l of formaldehyde solution in relation to various inactivation conditions.

### Example 3: Immunogenicity of Inactivated FCV virions

FCV 431 strains were cultured, inactivated and stabilized essentially as described in Example 1. This viral suspension was separated by size exclusion chromatography into two fractions containing, respectively, the virion (also named viral p66 fraction) and the soluble p66 protein (also named soluble p66 fraction). After separation, the two fractions were stored at 5°C.

A total of 12 vaccines were prepared, containing 1 ml of the viral p66 fraction diluted or undiluted (1/1, 1/4 and 1/16) and formulated with 1 ml of PBS (phosphate-buffered saline; without calcium and without magnesium); 1 ml of the viral p66 fraction diluted or undiluted (1/1, 1/4 and 1/16) and formulated with 1 ml of an oil-in-water emulsion (paraffin oil, fatty alcohol ethers and polyols, polyoxyethylene fatty acids); 1 ml of the soluble p66 fraction diluted or undiluted (1/1, 1/4 and 1/16) and formulated with the PBS; 1 ml of the soluble p66 fraction diluted or undiluted (1/1, 1/4 and 1/16) and formulated with the oil-water-emulsion.

The undiluted vaccines contain approximately the quantity of protein corresponding to 10 ml of crude viral culture. The viral p66 fractions and the soluble p66 fractions were diluted by addition of PBS without calcium and without magnesium.

Twenty-four specific pathogen free (SPF) kittens, each approximately 8-9 weeks old, were randomized into 12 groups and administered subcutaneously twice, 28 days apart, with 2 ml of these vaccines. No effect was observed relating to the injected quantity of p66 protein. The following results are reflective of all cats that were challenged via the oronasal route with FCV 431 virulent strain on day 46 (0.25 ml per nostril and 0.5 ml orally, 10^{5.5} CCID₅₀/ml).

Clinical signs were observed during the two weeks post-challenge. The scoring used for the calculation of the clinical score is as follow:

**Table 3: Clinical Scores of Vaccinated Felines**

| **Parameters** | **Observation** | **Score** |
|---|---|---|
| Rectal temperature | T° < 39.5°C | 0 |
| | 39.5°C ≤ T° < 40°C | 1 |
| | T° ≥ 40°C | 2 |
| General body condition | Normal | 0 |
| | Depression | 1 |
| Oronasal ulceration | Absence | 0 |
| | Diameter < 5 mm | 1 |
| | Diameter from 5 to 10 mm | 2 |
| | Diameter > 10 mm | 3 |
| Rhinitis | Serous nasal discharge | 1 |
| | Muco-purulent nasal discharge | 2 |
| Conjunctivitis | Serous discharge | 1 |
| | Muco-purulent discharge | 2 |
| Gingivitis | Present | 1 |
| Limping | Present | 1 |

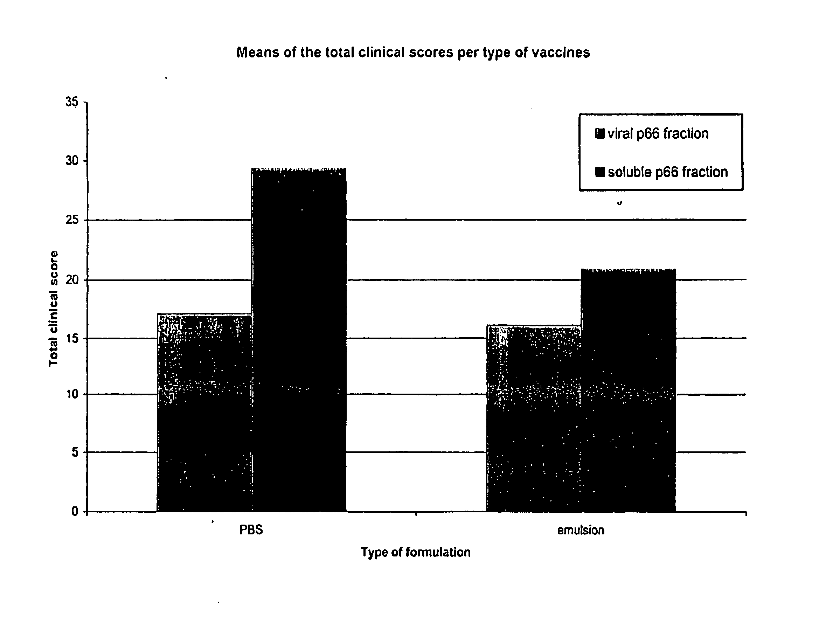

The total clinical score is the addition of all the clinical signs observed in a cat during the two weeks post-challenge. Means of the total clinical score per type of vaccines are indicated in the following table and in Figure 1.

**Table 4: Mean Total Clinical Score Per Type of Vaccine**

| | Viral p66 fraction | Soluble p66 fraction |
|---|---|---|
| PBS | 17.17 | 29.33 |
| Emulsion | 16.17 | 20.83 |

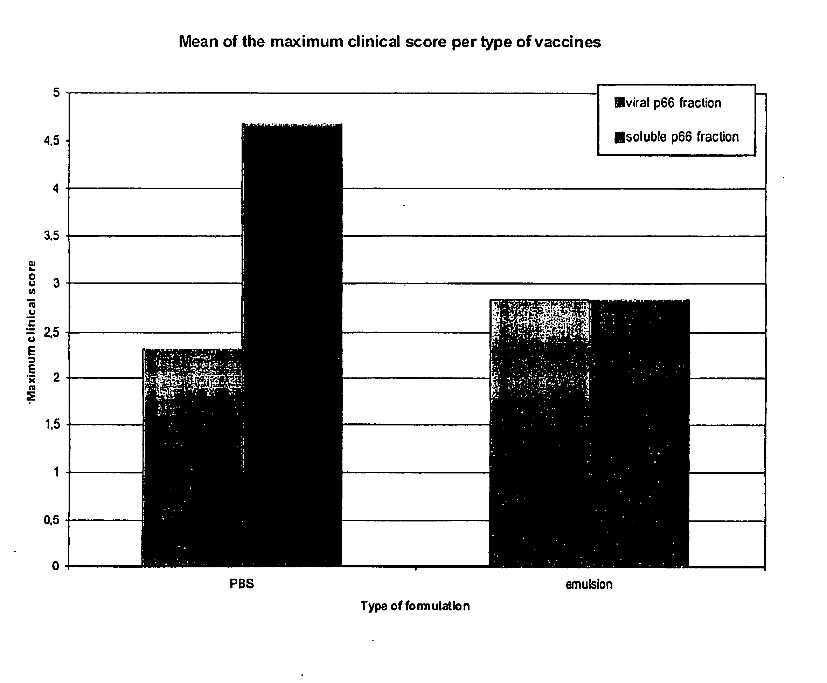

The maximum clinical score is the highest clinical score obtained for an observation on a cat during the two weeks post-challenge. Means of the maximum clinical score per type of vaccines are indicated in the following table and in Figure 2.

**Table 5: Mean Maximum Clinical Score Per Type of Vaccine**

| | Viral p66 fraction | Soluble p66 fraction |
|---|---|---|
| PBS | 2.33 | 4.67 |
| Emulsion | 2.83 | 2.83 |

There was a significant difference between means of the maximum clinical score obtained for viral p66 fraction/PBS and soluble p66 fraction/PBS groups (P-value, p=0.0495).

These results demonstrate that the inactivated virions maintain immunogenicity in absence of adjuvant. In the contrary, soluble p66 proteins induce a good immunogenic response only in presence of adjuvant.

### Example 4: Vaccination of Cats with Inactivated FCV

FCV 431 was cultured, inactivated with ethylenimine and stabilized by action of formaldehyde essentially as described in Example 1. The same procedure was applied for the FCV G1 strain.

A combined vaccine (vaccine A) comprised 5.60 log₁₀ CCID₅₀ per dose of attenuated feline rhinotracheitis virus (FHV-1), 4.17 log₁₀ CCID₅₀ per dose of attenuated feline panleucopenia virus (FPV), 8.29 log₁₀ CCID₅₀ per dose of canarypox vCP97 recombinant vector expressing *env* and *gag*/*pol* FeLV genes (see US-A-5.753.103), 4.8 log₁₀ ELD₅₀ per dose of attenuated Chlamydia, 8.7 log₁₀ CCID₅₀ per dose of inactivated and stabilized FCV 431 (equivalent titer of FCV before inactivation), 8.7 log₁₀ CCID₅₀ per dose of inactivated and stabilized FCV G1 (equivalent titer of FCV before inactivation), and physiological water.

Eighteen specific pathogen free kittens between 8 and 9 weeks old were randomized in 3 groups (6 kittens per group). The kittens of the group A were vaccinated with one dose of the vaccine A. The kittens of the group B were vaccinated with a mixture of a commercial vaccine containing in a freeze-drying form, a modified live FCV F9 strain, a modified live FHV-1 and a modified live FPV, and of a commercial vaccine containing an inactivated FeLV and Carbopol® adjuvant, this vaccine acting as diluent for the freeze-dried commercial vaccine. The kittens of the group C remained not vaccinated as control. The vaccine was administered twice, 28 days, apart, by subcutaneous route. Four weeks after the last vaccination, all cats were challenged via the oronasal route with FCV 255 virulent heterologous strain (Scott FW. Am. J. Vet. Res. 1977. 38(2). 229-34) (0.25 ml per nostril and 0.5 ml orally, 10^{8.2} CCID₅₀/ml).

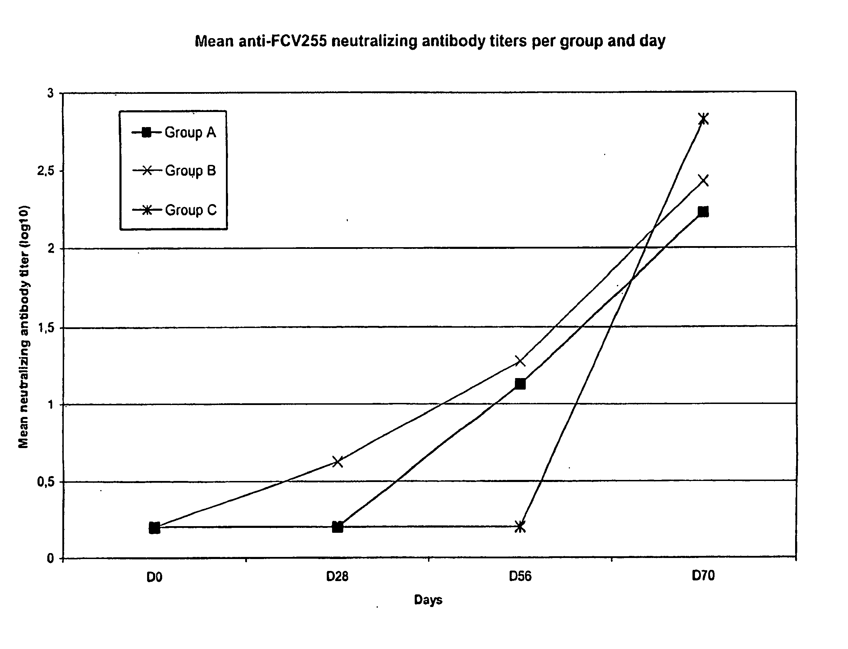

Anti-FCV255 neutralizing antibodies, animal weights, rectal temperatures, general conditions, general and local symptoms and viral excretions were observed during the two weeks post-challenge. The calicivirus antibody titers (expressed as log₁₀) are presented in the following table and in Figure 3.

**Table 6: Antibody Titers According to Group**

| | D0 | D28 | D56 | D70 |
|---|---|---|---|---|
| Group A | 0.20 | 0.20 | 1.13 | 2.23 |
| Group B | 0.20 | 0.62 | 1.28 | 2.43 |
| Group C | 0.20 | 0.20 | 0.20 | 2.83 |

On Day 0, antibody titers were negative in all groups.

After one injection of the vaccine, none of the vaccinated cats seroconverted except in group B. After the second injection, all cats had neutralizing antibody titers. Mean antibody titers at the time of challenge (D56) were not different between vaccinated groups (Tukey test) but were significantly higher than in the control group (ANOVA, p=0.0002).

The scoring used for the calculation of the global score is as follow:

**Table 7: Global Clinical Score of Vaccinated Cats**

| **Parameters** | **Observation** | **Score** |
|---|---|---|
| Rectal temperature | T° ≥ 39.5°C | 1 |
| | T° ≤ 37°C | 2 |
| General body condition* | Depression | 2 |
| | Death | 10 |
| Oronasal ulceration* | Small and few in number | 1 |
| | Large and numerous | 3 |
| Nasal discharge* | Slight | 1 |
| | Copious | 2 |
| Ocular discharge* | Present | 1 |
| Weight | Weight loss | 2 |

| | | |
|---|---|---|
| *The addition of the scores of the general body condition, oronasal ulceration, nasal discharge and ocular discharge represents the score of clinical symptoms as shown in Figure 4. | | |

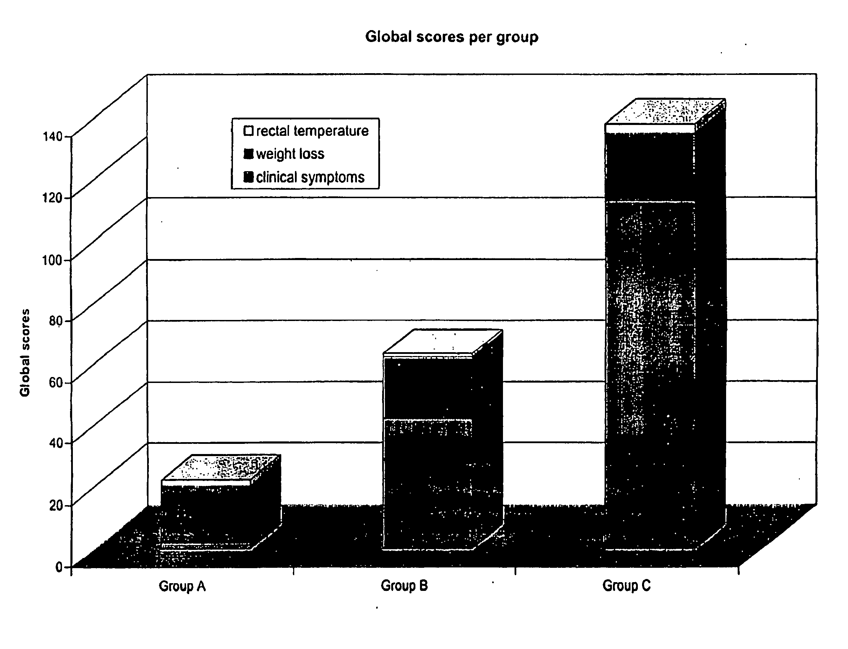

The clinical score results are presented in the following table and in Figure 4.

**Table 8: Clinical Scores of Vacciniated Cats**

| | Group A | Group B | Group C |
|---|---|---|---|
| Rectal temperature | 2 | 1 | 3 |
| Weight loss | 18 | 20 | 22 |
| Clinical symptoms | 4 | 43 | 116 |

All control cats, except one, exhibited typical FCV infection clinical symptoms. A pairwise comparison with the Tukey test showed that the group A was the only vaccinated group to be significantly different from the control group.

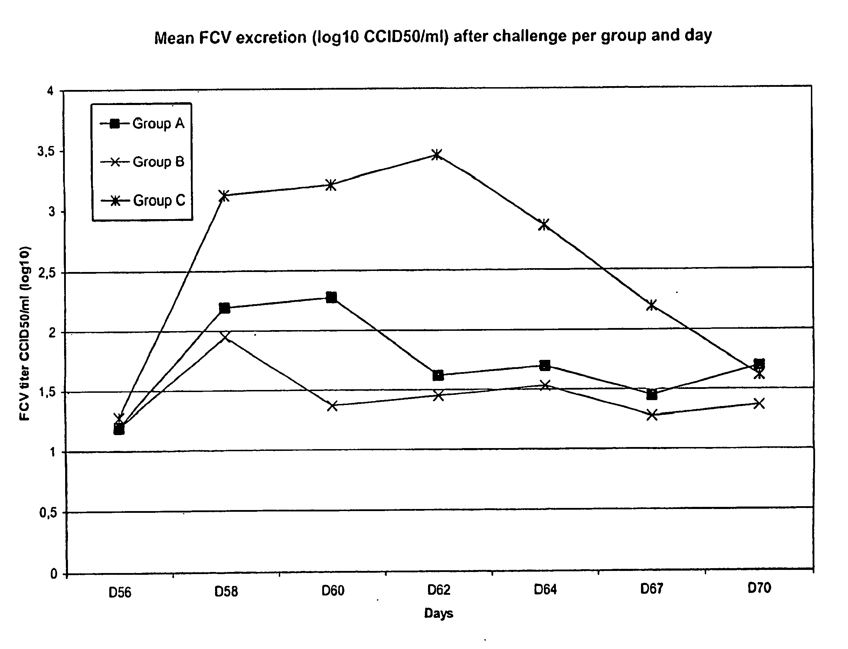

Pharyngeal swabs were collected and tested for viral excretion. The FCV excretion results (expressed as log10 CCID₅₀/ml) are presented in the following table and in Figure 5.

**Table 9: Excretion Levels of FCV in Vaccinated Cats**

| | D56 | D58 | D60 | D62 | D64 | D67 | D70 |
|---|---|---|---|---|---|---|---|
| Group A | 1.20 | 2.20 | 2.28 | 1.62 | 1.70 | 1.45 | 1.70 |
| Group B | 1.20 | 1.95 | 1.37 | 1.45 | 1.53 | 1.28 | 1.37 |
| Group C | 1.28 | 3.12 | 3.20 | 3.45 | 2.87 | 2.20 | 1.62 |

After challenge, viral excretion was observed in all groups but was reduced in vaccinated groups compared to the control group (ANOVA, p<0.00001). There was no difference between vaccinated groups (Tukey test).

Both vaccines protected cats against a heterologous FCV 255 challenge by strongly reducing clinical symptoms and viral excretion. The level of protection of the vaccines of the description was at least as good as that of the commercial modified live F9 vaccine. This demonstrates that an effective vaccination against FCV may be attained using an inactivated and stabilized FCV vaccine in the absence of adjuvant and that the level of protection is at least as good as that obtained with commercial live vaccines.

The in vivo compatibility between inactivated and stabilized FCV431/G1 and the other vaccine components (feline rhinotracheitis virus, feline infectious panleukopenia virus, feline leukemia virus and Chlamydia) has also been demonstrated.

## Claims

1. An inactivated, stabilized, non-adjuvanted immunogenic composition against feline calicivirus (FCV), comprising an FCV inactivated by one or more inactivating agents and stabilized by an aldehyde compound, wherein the aldehyde compound comprises a linear C1- C5 alkyl chain, and wherein the immunogenic composition is in admixture with an acceptable excipient or vehicle and wherein the inactivating agents are selected from the group consisting of ethyleneimine, acetylethyleneimine, propyleneimine, and β-propiolactone.

2. The composition of Claim 1, wherein the excipient or vehicle is veterinarily acceptable.

3. The composition of Claim 1, wherein the composition is freeze-dried and is in admixture with a freeze-drying excipient or vehicle.

4. The composition of any one of the preceding claims, wherein the inactivating agent is β-propiolactone.

5. The composition of any one of the preceding claims, wherein the inactivating agent is ethyleneimine and wherein the ethyleneimine is present from about 0.5 mM to about 20 mM.

6. The composition of any one of the preceding claims, wherein the inactivating agent is ethyleneimine and wherein the ethyleneimine is present from about 1 mM to about 10 mM.

7. The composition of Claim 1, wherein the aldehyde compound comprises a linear C1 alkyl chain and wherein the aldehyde compound comprises one aldehyde group.

8. The composition of Claim 1, wherein the aldehyde compound comprises a linear C2-C5 alkyl chain and wherein the aldehyde compound comprises two aldehyde groups.

9. The composition of Claim 8, wherein one of the two aldehyde groups is replaced by a ketone or an epoxy group.

10. The composition of Claim 1, wherein the aldehyde compound is selected from the group consisting of formaldehyde, glycidaldehyde, glutaraldehyde, glyoxal, or methylglyoxal.

11. The composition of Claim 10, wherein the aldehyde compound is formaldehyde and wherein the formaldehyde is present from about 0.05 g/l to about 0.8 g/l.

12. The composition of Claim 10, wherein the aldehyde compound is formaldehyde and wherein the formaldehyde is present from about 0.1 g/l to about 0.5 g/l.

13. The composition of Claim 1, further comprising a neutralizing compound, wherein the neutralizing compound comprises thiosulfate and cysteine.

14. The composition of Claim 1, further comprising at least one additional FCV strain, wherein at least one or both of the FCV strains is inactivated and stabilized.

15. The composition of Claim 14, wherein the at least one additional FCV strain is selected from the group consisting of FCV US 100869 (FCV PTA 5930), FCV F9, FCV 255, FCV 2280, FCV 431, FCV G1, FCV LLK, FCV KCD, FCV CFI, and FCV M8.

16. The composition of Claim 1, further comprising at least one non-FCV immunogen from a feline pathogen.

17. The composition of Claim 16, wherein the feline pathogen is selected from the group consisting of feline herpesvirus (FHV), feline leukemia virus (FeLV), feline panleukopenia virus (FPV), feline infectious peritonitis virus (FIPV), feline immunodeficiency virus (FIV), rabies virus, and feline Chlamydia.

18. The composition of Claim 16, wherein the at least one non-FCV immunogen from a feline pathogen comprises a live attenuated microorganism or a recombinant vector that expresses at least one immunogen from a feline pathogen.

19. A process for inactivating and stabilizing FCV, comprising the steps of reacting FCV with an inactivating agent and an aldehyde compound, wherein the aldehyde compound comprises a linear C1-C5 alkyl chain, and wherein the inactivating agent is selected from the group consisting of ethyleneimine, acetylethyleneimine, propyleneimine, and β-propiolactone, and recovering the inactivated and stabilized FCV.

20. The process of Claim 19, wherein the inactivating agent is β-propiolactone.

21. The process of Claim 19, wherein the inactivating agent is ethyleneimine and wherein the ethyleneimine is present from about 0.5 mM to about 20 mM.

22. The process of Claim 19, wherein the inactivating agent is ethyleneimine and wherein the ethyleneimine is present from about 1 mM to about 10 mM.

23. The process of Claim 19, wherein the aldehyde compound comprises a linear C1 alkyl chain and wherein the aldehyde compound comprises one aldehyde group.

24. The process of Claim 19, wherein the aldehyde compound comprises a linear C2-C5 alkyl chain and wherein the aldehyde compound comprises two aldehyde groups.

25. The process of Claim 24, wherein one of the two aldehyde groups is replaced by a ketone or an epoxy group.

26. The process of Claim 19, wherein the aldehyde compound is selected from the group consisting of formaldehyde, glycidaldehyde, glutaraldehyde, glyoxal, or methylglyoxal.

27. The process of Claim 26, wherein the aldehyde compound is formaldehyde and wherein the formaldehyde is present from about 0.05 g/l to about 0.8 g/l.

28. The process of Claim 26, wherein the aldehyde compound is formaldehyde and wherein the formaldehyde is present from about 0.1 g/l to about 0.5 g/l.

29. The process of Claim 19, further comprising the step of reacting the inactivated and stabilized FCV with a neutralizing compound, wherein the neutralizing compound comprises thiosulfate and cysteine.

30. The process of Claim 19, wherein the inactivated and stabilized FCV is recovered by size exclusion chromatography, ultracentrifugation, and selective precipitation.

31. The process of Claim 19, further comprising the step of freeze-drying the inactivated and stabilized FCV in a freeze-drying excipient.

32. A process for producing an inactivated, non-adjuvanted immunogenic composition, comprising mixing the inactivated and stabilized FCV of Claim 1 in an amount sufficient to induce an immune response to FCV with a veterinarily acceptable excipient or vehicle.

33. A process for producing an inactivated, non-adjuvanted immunogenic composition for long- term storage, comprising mixing the inactivated and stabilized FCV of Claim 1 in an amount sufficient to induce an immune response to FCV with a freeze-drying excipient and freezing the composition.

34. The inactivated, stabilized, non-adjuvanted immunogenic composition of Claim 1 for use in inducing an immune response in a Felidae against FCV..

35. The inactivated, stabilized, non-adjuvanted immunogenic composition of Claim 17 for use in inducing an immune response in a Felidae against FCV.

36. A combination comprising the inactivated, stabilized non-adjuvanted immunogenic composition of Claim 1 and at least one non-FCV immunogen from another feline pathogen for use in inducing an immune response in a Felidae against FCV and at least one non- FCV immunogen from a feline pathogen.

37. The combination according to Claim 36, wherein the at least one additional feline pathogen is selected from the group consisting of feline herpesvirus (FHV), feline leukemia virus (FeLV), feline panleukopenia virus (FPV), feline infectious peritonitis virus (FIPV), feline immunodeficiency virus (FIV), rabies virus, and feline Chlamydia

38. The combination according to Claim 36, wherein the at least one non-FCV immunogen from a feline pathogen comprises a live attenuated microorganism or a recombinant vector that expresses at least one immunogen from a feline pathogen.

## Patentansprüche

1. Inaktivierte, stabilisierte, nicht mit einem Hilfsstoff versetzte, immunogene Zusammensetzung gegen das Feline Calicivirus (FCV), umfassend ein FCV, das durch ein oder mehrere inaktivierende Agenzien inaktiviert ist und durch eine Aldehydverbindung stabilisiert ist, wobei die Aldehydverbindung eine lineare C1-C5-Alkylkette umfasst und wobei die immunogene Zusammensetzung im Gemisch mit einem akzeptablen Exzipienten oder Vehikel vorliegt und wobei die inaktivierenden Agenzien aus der Gruppe ausgewählt sind, die aus Ethylenimin, Acetylethylenimin, Propylenimin und β-Propiolacton besteht.

2. Zusammensetzung nach Anspruch 1, wobei der Exzipient oder das Vehikel tierärztlich akzeptabel ist.

3. Zusammensetzung nach Anspruch 1, wobei die Zusammensetzung gefriergetrocknet ist und im Gemisch mit einem gefriertrocknenden Exzipienten oder Vehikel vorliegt.

4. Zusammensetzung nach einem der vorherigen Ansprüche, wobei das inaktivierende Agens β-Propiolacton ist.

5. Zusammensetzung nach einem der vorherigen Ansprüche, wobei das inaktivierende Agens Ethylenimin ist und wobei das Ethylenimin in einer Konzentration von etwa 0,5 mM bis etwa 20 mM vorliegt.

6. Zusammensetzung nach einem der vorherigen Ansprüche, wobei das inaktivierende Agens Ethylenimin ist und wobei das Ethylenimin in einer Konzentration von etwa 1 mM bis etwa 10 mM vorliegt.

7. Zusammensetzung nach Anspruch 1, wobei die Aldehydverbindung eine lineare C1-Alkylkette umfasst und wobei die Aldehydverbindung eine Aldehydgruppe umfasst.

8. Zusammensetzung nach Anspruch 1, wobei die Aldehydverbindung eine lineare C2-C5-Alkylkette umfasst und wobei die Aldehydverbindung zwei Aldehydgruppen umfasst.

9. Zusammensetzung nach Anspruch 8, wobei eine der zwei Aldehydgruppen durch ein Keton oder eine Epoxygruppe ersetzt ist.

10. Zusammensetzung nach Anspruch 1, wobei die Aldehydverbindung aus der Gruppe ausgewählt ist, die aus Formaldehyd, Glycidaldehyd, Glutaraldehyd, Glyoxal oder Methylglyoxal besteht.

11. Zusammensetzung nach Anspruch 10, wobei die Aldehydverbindung Formaldehyd ist und wobei das Formaldehyd in einer Konzentration von etwa 0,05 g/l bis etwa 0,8 g/l vorliegt.

12. Zusammensetzung nach Anspruch 10, wobei die Aldehydverbindung Formaldehyd ist und wobei das Formaldehyd in etwa 0,1 g/l bis etwa 0,5 g/l vorliegt.

13. Zusammensetzung nach Anspruch 1, die weiterhin eine neutralisierende Verbindung umfasst, wobei die neutralisierende Verbindung Thiosulfat und Cystein umfasst.

14. Zusammensetzung nach Anspruch 1, die weiterhin mindestens einen zusätzlichen FCV-Stamm umfasst, wobei mindestens einer oder beide der FCV-Stämme inaktiviert und stabilisiert ist.

15. Zusammensetzung nach Anspruch 14, wobei der mindestens eine zusätzliche FCV-Stamm aus der Gruppe ausgewählt ist, die aus FCV100869 US (FCV PTA 5930), FCV F9, FCV 255, FCV 2280, FCV 431, FCV G1, FCV LLK, FCV KCD, FCV CFI und FCV M8 besteht.

16. Zusammensetzung nach Anspruch 1, die weiterhin mindestens ein Nicht-FCV-Immunogen eines Katzenpathogens umfasst.

17. Zusammensetzung nach Anspruch 16, wobei das Katzenpathogen aus der Gruppe ausgewählt ist, die aus dem Felinen Herpesvirus (FHV), Felinen Leukämievirus (FeLV), Felinen Panleukopenie-Virus (FPV), Felinen Infektiösen Peritonitis-Virus (FIPV), Felinen Immunschwächevirus (FIV), Rabiesvirus und felinen Chlamydien besteht.

18. Zusammensetzung nach Anspruch 16, wobei mindestens ein Nicht-FCV-Immunogen eines Katzenpathogens einen lebenden attenuierten Mikroorganismus oder einen rekombinanten Vektor umfasst, der mindestens ein Immunogen eines Katzenpathogens exprimiert.

19. Verfahren zur Inaktivierung und Stabilisierung von FCV, das die Schritte umfasst:
Umsetzen von FCV mit einem inaktivierenden Agens und einer Aldehydverbindung, wobei die Aldehydverbindung eine lineare Cl-C5-Alkylkette umfasst, und wobei das inaktivierende Agens aus der Gruppe ausgewählt ist, die aus Ethylenimin, Acetylethylenimin, Propylenimin und β-Propiolacton besteht, und
Gewinnen des inaktivierten und stabilisierten FCVs.

20. Verfahren nach Anspruch 19, wobei das inaktivierende Agens β-Propiolacton ist.

21. Verfahren nach Anspruch 19, wobei das inaktivierende Agens Ethylenimin ist und wobei das Ethylenimin in einer Konzentration von etwa 0,5 mM bis etwa 20 mM vorliegt.

22. Verfahren nach Anspruch 19, wobei das inaktivierende Agens Ethylenimin ist und wobei das Ethylenimin in einer Konzentration von etwa 1 mM bis etwa 10 mM vorliegt.

23. Verfahren nach Anspruch 19, wobei die Aldehydverbindung eine lineare C1-Alkylkette umfasst und wobei die Aldehydverbindung eine Aldehydgruppe umfasst.

24. Verfahren nach Anspruch 19, wobei die Aldehydverbindung eine lineare C2-C5-Alkylkette umfasst und wobei die Aldehydverbindung zwei Aldehydgruppen umfasst.

25. Verfahren nach Anspruch 24, wobei eine der zwei Aldehydgruppen durch ein Keton oder eine Epoxygruppe ersetzt ist.

26. Verfahren nach Anspruch 19, wobei die Aldehydverbindung aus der Gruppe ausgewählt ist, die aus Formaldehyd, Glycidaldehyd, Glutaraldehyd, Glyoxal oder Methylglyoxal besteht.

27. Verfahren nach Anspruch 26, wobei die Aldehydverbindung Formaldehyd ist und wobei das Formaldehyd in einer Konzentration von etwa 0,05 g/l bis etwa 0,8 g/l vorliegt.

28. Verfahren nach Anspruch 26, wobei die Aldehydverbindung Formaldehyd ist und wobei das Formaldehyd in einer Konzentration von etwa 0,1 g/l bis etwa 0,5 g/l vorliegt.

29. Verfahren nach Anspruch 19, das weiterhin den Schritt des Umsetzens des inaktivierten und stabilisierten FCVs mit einer neutralisierenden Verbindung umfasst, wobei die neutralisierende Verbindung Thiosulfat und Cystein umfasst.

30. Verfahren nach Anspruch 19, wobei das inaktivierte und stabilisierte FCV durch Ausschlusschromatographie, Ultrazentrifugation und selektive Präzipitation gewonnen wird.

31. Verfahren nach Anspruch 19, das weiterhin den Schritt des Gefriertrocknens des inaktivierten und stabilisierten FCVs in einem gefriertrocknenden Exzipienten umfasst.

32. Verfahren zur Herstellung einer inaktivierten, nicht mit einem Hilfsstoff versetzten immunogenen Zusammensetzung, umfassend das Mischen des inaktivierten und stabilisierten FCVs nach Anspruch 1 in einer Menge, die ausreicht, um eine Immunantwort auf FCV zu induzieren, mit einem tierärztlich akzeptablen Exzipienten oder Vehikel.

33. Verfahren zur Herstellung einer inaktivierten, nicht mit einem Hilfsstoff versetzten immunogenen Zusammensetzung zur langfristigen Lagerung, umfassend das Mischen des inaktivierten und stabilisierten FCVs nach Anspruch 1 in einer Menge, die ausreicht, um eine Immunantwort auf FCV zu induzieren, mit einem gefriertrocknenden Exzipienten und das Einfrieren der Zusammensetzung.

34. Inaktivierte, stabilisierte, nicht mit einem Hilfsstoff versetzte immunogene Zusammensetzung nach Anspruch 1 zur Verwendung für die Induktion einer Immunantwort auf FCV in Feliden.

35. Inaktivierte, stabilisierte, nicht mit einem Hilfsstoff versetzte immunogene Zusammensetzung nach Anspruch 17 zur Verwendung für die Induktion einer Immunantwort auf FCV in Feliden.

36. Kombination, umfassend die inaktivierte, stabilisierte, nicht mit einem Hilfsstoff versetzte immunogene Zusammensetzung nach Anspruch 1 und mindestens ein Nicht-FCV-Immunogen eines anderen Katzenpathogens zur Verwendung für die Induktion einer Immunantwort gegen FCV und mindestens ein Nicht-FCV-Immunogen eines Katzenpathogens in Feliden.

37. Kombination nach Anspruch 36, wobei das mindestens eine zusätzliche Katzenpathogen aus der Gruppe ausgewählt ist, die aus dem Felinen Herpesvirus (FHV), Felinen Leukämievirus (FeLV), Felinen Panleukopenie-Virus (FPV), Felinen Infektiösen Peritonitis-Virus (FIPV), Felinen Immunschwächevirus (FIV), Rabiesvirus und felinen Chlamydien besteht.

38. Kombination nach Anspruch 36, wobei das mindestens eine Nicht-FCV-Immunogen eines Katzenpathogens einen lebenden attenuierten Mikroorganismus oder einen rekombinanten Vektor umfasst, der mindestens ein Immunogen eines Katzenpathogens exprimiert.

## Revendications

1. Composition immunogène inactivée, stabilisée et sans adjuvant pour lutter contre le calicivirus félin (FCV), comprenant un FCV inactivé par un ou plusieurs agents d'inactivation et stabilisé par un composé aldéhydique, dans laquelle le composé aldéhydique comprend une chaîne alkyle linéaire en C₁ à C₅, et dans laquelle la composition immunogène est en mélange avec un excipient ou véhicule acceptable et dans laquelle les agents d'inactivation sont choisis dans le groupe comprenant l'éthylèneimine, l'acétyléthylèneimine, la propylèneimine et la β-propiolactone.

2. Composition selon la revendication 1, dans laquelle l'excipient ou le véhicule est acceptable d'un point de vue vétérinaire.

3. Composition selon la revendication 1, dans laquelle la composition est lyophilisée et est en mélange avec un excipient ou véhicule de lyophilisation.

4. Composition selon l'une quelconque des revendications précédentes, dans laquelle l'agent d'inactivation est la β-propiolactone.

5. Composition selon l'une quelconque des revendications précédentes, dans laquelle l'agent d'inactivation est l'éthylèneimine et dans laquelle l'éthylèneimine est présente en une quantité allant d'environ 0,5 mM à environ 20 mM.

6. Composition selon l'une quelconque des revendications précédentes, dans laquelle l'agent d'inactivation est l'éthylèneimine et dans laquelle l'éthylèneimine est présente en une quantité allant d'environ 1 mM à environ 10 mM.

7. Composition selon la revendication 1, dans laquelle le composé aldéhydique comprend une chaîne alkyle linéaire en C₁ et dans laquelle le composé aldéhydique comprend un groupe aldéhyde.

8. Composition selon la revendication 1, dans laquelle le composé aldéhydique comprend une chaîne alkyle linéaire en C₂ à C₅ et dans laquelle le composé aldéhydique comprend deux groupes aldéhydes.

9. Composition selon la revendication 8, dans laquelle un des deux groupes aldéhydes est remplacé par une cétone ou un groupe époxy.

10. Composition selon la revendication 1, dans laquelle le composé aldéhydique est choisi dans le groupe comprenant le formaldéhyde, le glycidaldéhyde, le glutaraldéhyde, le glyoxal ou le méthylglyoxal.

11. Composition selon la revendication 10, dans laquelle le composé aldéhydique est le formaldéhyde et dans laquelle le formaldéhyde est présent en une quantité allant d'environ 0,05 g/l à environ 0,8 g/l.

12. Composition selon la revendication 10, dans laquelle le composé aldéhydique est le formaldéhyde et dans laquelle le formaldéhyde est présent en une quantité allant d'environ 0,1 g/l à environ 0,5 g/l.

13. Composition selon la revendication 1, comprenant en outre un composé de neutralisation, dans laquelle le composé de neutralisation comprend le thiosulfate et la cystéine.

14. Composition selon la revendication 1, comprenant en outre au moins une souche supplémentaire de FCV, dans laquelle au moins une des souches de FCV ou les deux souches de FCV sont inactivées et stabilisées.

15. Composition selon la revendication 14, dans laquelle la au moins une souche supplémentaire de FCV est choisie dans le groupe comprenant le FCV US 100869 (FCV PTA 5930), le FCV F9, le FCV 255, le FCV 2280, le FCV 431, le FCV G1, le FCV LLK, le FCV KCD, le FCV CFI et le FCV M8.

16. Composition selon la revendication 1, comprenant en outre au moins un immunogène non-FCV provenant d'un pathogène félin.

17. Composition selon la revendication 16, dans laquelle le pathogène félin est choisi dans le groupe comprenant l'herpès virus félin (FHV), le virus de la leucémie féline (FeLV), le virus de la panleucopénie féline (FPV), le virus de la péritonite infectieuse féline (FIPV), le virus de l'immunodéficience féline (FIV), le virus de la rage et le Chlamydia félin.

18. Composition selon la revendication 16, dans laquelle le au moins un immunogène non FCV provenant d'un pathogène félin comprend un micro-organisme vivant atténué ou un vecteur recombinant qui exprime au moins un immunogène provenant d'un pathogène félin.

19. Procédé d'inactivation et de stabilisation de FCV, comprenant les étapes de réaction du FCV avec un agent d'inactivation et un composé aldéhydique, dans lequel le composé aldéhydique comprend une chaîne alkyle linéaire en C₁ à C₅ et dans lequel l'agent d'inactivation est choisi dans le groupe comprenant l'éthylèneimine, l'acétyléthylèneimine, la propylèneimine et la β-propiolactone, et de récupération du FCV inactivé et stabilisé.

20. Procédé selon la revendication 19, dans lequel l'agent d'inactivation est la β-propiolactone.

21. Procédé selon la revendication 19, dans lequel l'agent d'inactivation est l'éthylèneimine et dans lequel l'éthylèneimine est présente en une quantité allant d'environ 0,5 mM à environ 20 mM.

22. Procédé selon la revendication 19, dans lequel l'agent d'inactivation est l'éthylèneimine et dans lequel l'éthylèneimine est présente en une quantité allant d'environ 1 mM à environ 10 mM.

23. Procédé selon la revendication 19, dans lequel le composé aldéhydique comprend une chaîne alkyle linéaire en C₁ et dans lequel le composé aldéhydique comprend un groupe aldéhyde.

24. Procédé selon la revendication 19, dans lequel le composé aldéhydique comprend une chaîne alkyle linéaire en C₂ à C₅ et dans lequel le composé aldéhydique comprend deux groupes aldéhydes.

25. Procédé selon la revendication 24, dans lequel un des deux groupes aldéhydes est remplacé par une cétone ou un groupe époxy.

26. Procédé selon la revendication 19, dans lequel le composé aldéhydique est choisi dans le groupe comprenant le formaldéhyde, le glycidaldéhyde, le glutaraldéhyde, le glyoxal ou le méthylglyoxal.

27. Procédé selon la revendication 26, dans lequel le composé aldéhydique est le formaldéhyde et dans lequel le formaldéhyde est présent en une quantité allant d'environ 0,05 g/l à environ 0,8 g/l.

28. Procédé selon la revendication 26, dans lequel le composé aldéhydique est le formaldéhyde et dans lequel le formaldéhyde est présent en une quantité allant d'environ 0,1 g/l à environ 0,5 g/l.

29. Procédé selon la revendication 19, comprenant en outre l'étape de réaction du FCV inactivé et stabilisé avec un composé de neutralisation, dans lequel le composé de neutralisation comprend le thiosulfate et la cystéine.

30. Procédé selon la revendication 19, dans lequel le FCV inactivé et stabilisé est récupéré par chromatographie d'exclusion stérique, ultracentrifugation et précipitation sélective.

31. Procédé selon la revendication 19, comprenant en outre l'étape de lyophilisation du FCV inactivé et stabilisé dans un excipient de lyophilisation.

32. Procédé de production d'une composition immunogène inactivée et sans adjuvant, comprenant le mélange du FCV inactivé et stabilisé selon la revendication 1 en une quantité suffisante pour induire une réponse immunitaire dirigée contre le FCV avec un excipient ou véhicule est acceptable d'un point de vue vétérinaire.

33. Procédé de production d'une composition immunogène inactivée et sans adjuvant pour un stockage de longue durée, comprenant le mélange du FCV inactivé et stabilisé selon la revendication 1 en une quantité suffisante pour induire une réponse immunitaire dirigée contre le FCV avec un excipient de lyophilisation et la congélation de la composition.

34. Composition immunogène inactivée, stabilisée et sans adjuvant selon la revendication 1, utilisable pour induire une réponse immunitaire chez un félidé dirigée contre un FCV.

35. Composition immunogène inactivée, stabilisée et sans adjuvant selon la revendication 17, utilisable pour induire une réponse immunitaire chez un félidé dirigée contre un FCV.

36. Combinaison comprenant la composition immunogène inactivée, stabilisée et sans adjuvant selon la revendication 1 et au moins un immunogène non FCV provenant d'un autre pathogène félin utilisable pour induire une réponse immunitaire chez un félidé dirigée contre un FCV et au moins un immunogène non FCV provenant d'un pathogène félin.

37. Combinaison selon la revendication 36, dans laquelle le au moins un pathogène félin supplémentaire est choisi dans le groupe comprenant l'herpès virus félin (FHV), le virus de la leucémie féline (FeLV), le virus de la panleucopénie féline (FPV), le virus de la péritonite infectieuse féline (FIPV), le virus de l'immunodéficience féline (FIV), le virus de la rage et le Chlamydia félin.

38. Combinaison selon la revendication 36, dans laquelle le au moins un immunogène non FCV provenant d'un pathogène félin comprend un micro-organisme vivant atténué ou un vecteur recombinant qui exprime au moins un immunogène provenant d'un pathogène félin.
